Europäisches Patentamt

European Patent Office

Office européen des brevets

(11) Publication number: **0 276 120 B1**

## EUROPEAN PATENT SPECIFICATION

(45) Date of publication of patent specification: **08.04.92**  (51) Int. Cl.[5]: **A61K 37/66**

(21) Application number: **88300407.9**

(22) Date of filing: **19.01.88**

(54) **Treatment of certain leukemias with a combination of gamma interferon and alpha interferon.**

(30) Priority: **20.01.87 US 4731**

(43) Date of publication of application:
**27.07.88 Bulletin 88/30**

(45) Publication of the grant of the patent:
**08.04.92 Bulletin 92/15**

(84) Designated Contracting States:
**AT BE CH DE ES FR GB GR IT LI LU NL SE**

(56) References cited:
**EP-A- 0 087 686**
**EP-A- 0 107 498**
**EP-A- 0 225 759**

**JOURNAL OF BIOLOGICAL RESPONSE MODIFIERS Vol. 6, no. 2, 1987, pages 141-153, New York, US; H.R. HUBBELL et al.: "Synergistic antiproliferative effect of recombinant alpha-interferon with gamma-interferon"**

**JOURNAL OF IMMUNOLOGY vol. 129, no. 1, July 1982, pages 76-80, Baltimore, US; G.R.KLIMPEL et al.: "Gamma-interferon and alpha/beta interferon supress murine myeloid colony formation(CFU-C)"**

(73) Proprietor: **SCHERING CORPORATION
2000 Galloping Hill Road
Kenilworth New Jersey 07033(US)**

(72) Inventor: **Trotta, Paul Philip
405 Park Avenue
Rutherford New Jersey 07070(US)**

(74) Representative: **Ritter, Stephen David et al
Mathys & Squire 10 Fleet Street
London EC4Y 1AY(GB)**

Rank Xerox (UK) Business Services

**Description**

This invention relates to the manufacture of a medicament for combination therapies for inhibiting the proliferation of susceptible human lymphoma derived B-cells. More specifically, the manufacture of a medicament involving the use in combination of (a) recombinant DNA derived human gamma interferon (hIFN-y) sometimes referred to as human immune interferon and (b) recombinant DNA derived human alpha interferon, which in combination display greater antiproliferative effects against said leukemia cells than is expected from their individual activities.

Gamma interferon has a number of characteristics known in the art that differentiate it from alpha and beta interferons. Among these differences are antigenic distinctiveness and higher levels of immunoregulatory and antitumor activity. Human gamma interferon may be produced by T-lymphocytes stimulated by mitogens or antigens to which they are sensitized. It may also be obtained through cloning and expression techniques now well known in the art.

Although the source of the human gamma interferon used in this invention is not critical, it is required that such hIFN-y be of a high purity material that is not contaminated by cell constituents or cell debris of the interferon-expressing cell. A preferred hIFN-$\gamma$ used in this invention is produced by recombinant DNA technology and then purified as taught in Japanese Patent Application No. 281376, filed December 27, 1984 and foreign counterparts thereof, e.g. PCT International Publication No. 8604067 published July 17. 1986. The purification process comprises adding one or more salts of zinc or copper and polyethyleneimine in the extraction. More particularly, it comprises suspending the culture cells of a recombinant microorganism in a buffer solution containing one or more salts of zinc or copper, e.g. zinc chloride, zinc sulfate, zinc acetate, zinc acetylacetonate and copper sulfate, in a range of about 0.5-5 mM in the case of zinc salts and 0.01-3 mM in the case of copper salts, disrupting the cells, then adding polyethyleneimine to the centrifuged supernatant, e.g. to a final concentration of 0.5-1.1%, and subsequently purifying by a conventional method, e.g. combining several chromatographic stages and dialysis.

Human gamma interferon can be made, for example, by the procedures disclosed in Gray, et al., Nature, 295, 503-508 (1982) and Epstein, Nature, 295, 453-454 (1982).

Human alpha interferon is a naturally occurring mixture of at least eleven compounds including those designated alpha-1 interferon and alpha-2 interferon. Alpha interferon exhibiting biological properties similar to those of naturally occurring human or leukocyte interferon can be made by recombinant methods.

A number of alpha interferon species or components are known and are usually designated by a numeral after the Greek letter alpha, and all are contemplated for use in this invention. Thus, the species designated "human alpha-1 interferon" is contemplated for use in this invention and "human alpha-2 interferon" which under USAN, is designated Interferon Alfa-2b, is also contemplated for use in this invention. "Human interferon alfa-2b" is used herein when referring to human alpha-2 interferon. Interferon alfa-2b is the preferred species of human interferon alfa-2.

Human interferon alfa-2 can be produced in bacteria using recombinant techniques. In addition, human interferon alfa-2b may be prepared by recombinant-DNA methods disclosed by Nagata et al., Nature, 284, 316-320 (1980), European Patent 32,134 and U.S. Patent No. 4,289,690. Various interferon alfa-2 species are disclosed in U.S. Patent 4,503,035. The preferred human interferon alfa-2b used in this invention is also denoted "hIFN-$\alpha$2b".

To date, much has been learned about the effect of the interferons on cell proliferation.

Borden et al., Progress in Hematology, Vol XII, Brown, E.B., editor, pp 299-339 (1981) found that interferons inhibit proliferation of normal and transformed cells in vitro.

Gresser et al., Biochim. Biophys. Acta, 516, 231-247 (1978) and Cancer-A Comprehensive Treatise, Becker, F. editor, Vol. 5, pp 521-571 (1977) used murine systems and demonstrated that relatively impure interferon in vitro results in a decreased rate of cell proliferation, lower cell-saturation densities and decreased colony formation in agarose. Inhibitory effects were observed using mouse L929, L1210, Ehrlich ascites, primary embryo and primary weanling kidney cells.

Different types of cells differ in sensitivity to interferons, and cell inhibitory concentrations of interferons can vary widely, i.e. from 10-1000 units, see Borden et al. supra. Among the tumor cells of established cell lines studied in vitro, Einhorn et al., Human Interferon Production and Clinical Use, Stinebring et al., editors, pp 159-174 (1977), found lymphoid leukemias to be sensitive to interferons. The interferons used in these experiments were unpurified materials and it is not clear if the impurities affected the test results.

European Patent Application, Publication No. 0107498, OCtober 24, 1983 discloses combinations of purified human gamma interferon and human alpha interferon made by recombinant methods which exhibit activities higher than could have been fairly predicted based on their respective activities when tested alone. The tests were conducted using the human melanoma cell line Hs294T. However, there is no disclosure that leukemias are susceptible to such treatment.

According to the invention, there is provided the use of recombinant human alpha interferon in the manufacture of a pharmaceutical composition for use in inhibiting the proliferation of susceptible human lymphoma derived B cell leukemia cells by a combination therapy involving simultaneously or sequentially treating such cells with an effective amount of a combination of recombinant human alpha interferon and recombinant human gamma interferon.

Also provided is the use of recombinant human gamma interferon in the manufacture of a pharmaceutical composition for use in inhibiting the proliferation of susceptible human lymphoma derived B cell leukemia cells by a combination therapy involving simultaneously or sequentially treating such cells with an effective amount of a combination of recombinant human alpha interferon and recombinant human gamma interferon.

The aforementioned uses are applicable to provide therapies for inhibiting the proliferation of susceptible human lymphoma derived B-cell leukemia cells with a cell proliferation inhibiting amount of a combination of recombinant human gamma interferon and recombinant human alpha interferon (preferably hIFN- 2b) sufficient to inhibit the proliferation of the cells thereof. When the susceptible leukemia cells are in an animal, including humans, the preferred mode of administration is parenteral, i.e. intravenous, intramuscular, subcutaneous and intraperitoneal, although enteral modes, e.g.; oral, nasal, and the like, as well as topical modes of administration are also comtemplated. The interferons can be administered simultaneously or sequentially.

MATERIALS AND METHODS

In order to determine the antiproliferative effects against human T-cells and human B-cell lines, it is possible to run tests on cell cultures using suspension cultures of the cell lines propagated in medium RPMI 1640 supplemented with 10% heat-inactivated fetal bovine serum, 5 $\mu$M of 2-mercaptoethanol, 100 units/ml penicillin, 100 $\mu$g/ml of streptomycin, and 50 $\mu$g/ml of gentamicin as described in Moore, G.E., Human Cells in vitro, Fogh, J. editor, pp 299-331 (1975) and Ohnuma et al., J. Natl. Cancer Inst. 60 1117-20 (1978).

The cell lines used are readily available. The human T-cell lines used were MOLT-3 (ATCC CRL 1552), MOLT-4 (ATTC CRL 1582), HSB-2 (Tumor Institute, University of Alabama at Birmingham AL) and RPMI 8402 (Mount Sinai School of Medicine, New York, NY); the human B-cell lines used were BALM-2 and DND-39A (Mount Sinai School of Medicine, NY, NY), Daudi (ATTC CCL 213) and SB (Tumor Institute, University of Alabama at Birmingham, AL).

The above cultures were reseeded at 3- and 4-day intervals at 2.0-3.0 $\times$ $10^5$ cells/ml in 75 cm$^2$ tissue culture flasks in a total volume of 100 ml per flask. The cell populations exponentially proliferate between cell densities of 2.0 $\times$ $10^5$ to 1.5 $\times$ $10^6$ cells/ml.

Interferons

The hIFN-$\alpha$2b used in the tests had a specific activity of 1.5 $\times$ $10^8$ International Reference Units (IRU) per mg of protein and the recombinant human gamma interferon had a specific activity of 2 $\times$ $10^6$ IRU/mg of protein.

Experimental Procedure

The cell lines were propagated in RPMI 1640 medium supplemented with 10% heat inactivated fetal bovine serum as described above. The cell cultures were initiated at a cell density of 2.0 $\times$ $10^5$ cells/ml in a total volume of 24 ml in 25 cm$^2$ Corning tissue culture flasks and incubated at 37°C as stationary suspension cultures. The interferons were diluted to designated concentrations in RPMI 1640 medium supplemented with 10% heat inactivated fetal bovine serum. Adriamycin used as a positive control, was dissolved in sterile deionized water and diluted in cell culture medium supplemented with 10% heat inactivated fetal bovine serum to a final concentration of 0.2 $\mu$g/ml which is the concentration comparable to drug levels in the tissues of patients and experimental animals during therapy.

In the experiments, 24 hours after the cell cultures were initiated, the proliferating cell populations were exposed to different concentrations of human alpha-2 interferon, preferably, interferon alfa-2b, for 24 and 48 hours, e.g. 0.0066, 0.066, 0.66 and 6.66 $\mu$g/ml. At 24 and 48 hours, samples were withdrawn from the samples treated with test drug, untreated control and treated with control drug and counted in a hemacytometer. Viable cell (trypan-blue-excluding cells) counts were used in determining the effect of human alfa-2b interferon and adriamycin on the proliferation of the cultures.

In other experiments, either human alfa-2b interferon or human gamma interferon at various concentrations, e.g. 0.0066, 0.066, 0.66 and 6.66 µg/ml, was added at the time of initiation of cell cultures, and the number of cells monitored as a function of time through 96 hours, i.e. at 24, 48, 72 and 96 hours. At each time interval, samples were withdrawn and counted in a hemacytometer. Interferon assay results are expressed as percent growth inhibition calculated according to the following equation:

$$\text{Percent Growth Inhibition} = 1.0 - \frac{NT_t - NC_o}{NC_t - NC_o} \times 100$$

$NT_t$ = Cell density of culture at time T.

$NC_t$ = Cell density of control culture at time T.

$NC_o$ = The initial cell density of the culture prior to addition of interferon or adriamycin.

The results expressed in %GI (Growth Inhibition) are summarized Tables I and II.

## TABLE 1

## Effect of Human alfa-2b. Interferon on

## Human B- and T-cell Leukemias

| LEUKEMIA CELL TYPE | | CONCENTRATIONS µG/ML, hIFN-α-2b | | | | ADR µG/ML |
|---|---|---|---|---|---|---|
| | | 0.0066 | 0.066 | 0.66 | 6.66 | 0.2 |
| Human B-Cell SB | % GI Hours | 60;47 24;48 | 60;47 24;48 | 60;47 24;48 | 60;47 24;48 | 100 24-48 |
| Human B-Cell DND-39A | % GI Hours | 0;19 24;49 | 0;19 24;48 | 0;42 24;48 | 85;42 24;48 | 100 24-48 |
| Human B-Cell BALM-2 | % GI Hours | 0;27 24;48 | 39;50 24;48 | 69;50 24;48 | 69;66 24;48 | 100 24-48 |
| Human T-Cell MOLT-4 | % GI Hours | 100;86 24;48 | 100;86 24;48 | 100;86 24;48 | 100;86 24;48 | 100 24-48 |
| Human T-Cell MOLT-3 | % GI Hours | 62;0 24;48 | 62;0 24;48 | 62;0 24;48 | 62;0 24;48 | 100 24-48 |
| Human B-Cell Daudi | % GI Hours | 0 24;48 | 0 24;48 | 0 24;48 | 0 24;48 | 100 24-48 |
| Human T-Cell HSB-2 | % GI Hours | 0 24;48 | 0 24;48 | 0 24;48 | 0 24;48 | 100 24-48 |
| Human T-Cell RPMI 8402 | % GI Hours | 0;42 24;48 | 0;42 24;48 | 0;42 24;48 | 0;42 24;48 | 100 24-48 |
| Human B-Cell Daudi | % GI Hours | 0;100 48;72-96 | 0;100 48;72-96 | 0;100 48;72-96 | 0;100 48;72-96 | 100;NVC 48;72-96 |

| LEUKEMIA CELL TYPE | | | CONCENTRATIONS NG/ML. hIFN-α-2b | | | ADR µG/ML |
|---|---|---|---|---|---|---|
| | | 0.006 | 0.066 | 0.66 | 6.66 | 0.2 |
| Human B-Cell Daudi | % GI Hours | 0 24;72;96 | 0;87;70 24;72;96 | 0;87;100 24;72;96 | 0;87;100 24;72;96 | 100 24;72;96 |
| Human T-Cell RPMI 8402 | % GI Hours | 0 48-96 | 0;46;25 48;72;96 | 0;46;53 48;72;96 | 0;46;78 48;72;96 | 100 48-96 |
| Human T-Cell MOLT-4 | % GI Hours | 0 24-96 | 0 24-96 | 0;36;28 24-48;72;96 | 0;52;44 24-48;72;96 | 100 24-96 |
| Human T-Cell MOLT-3 | % GI Hours | 0;26 24-48;72 | 0;26 24-48;72 | 0;26 24-48;72 | 0;55 24-48;72 | 100 24-72 |

NVC = No viable cells.
GI  = Growth Inhibition

TABLE II

Effect of Human Gamma Interferon
on Human B- and T-Cell Leukemias

| LEUKEMIA CELL TYPE | | | CONCENTRATIONS NG/ML, hIFN-γ | | | ADR µG/ML |
|---|---|---|---|---|---|---|
| | | 0.0066 | 0.066 | 0.66 | 6.66 | 0.2 |
| Human B-Cell SB | % GI Hours | 0 24;48 | 0 24;48 | 0 24;48 | 0 24;48 | 100 24-48 |
| Human T-Cell MOLT-4 | % GI Hours | 0 24;48 | 0 24;48 | 0 24;48 | 0 24;48 | 100 24-48 |
| Human B-Cell DND-39A | % GI Hours | 0;32 24-48;72 | 0;62;32 24;48;72 | 0;62;32 24;48;72 | 0;87;32 24;48;72 | 100 24-72 |
| Human T-Cell MOLT-3 | % GI Hours | 100;64;0 24;48;72 | 100;64;0 24;48;72 | 100;64;0 24;48;72 | 100;64;0 24;48;72 | 100 24-72 |
| Human B-Cell Daudi | % GI Hours | 0 48-72 | 0 48-72 | 0 48-72 | 0 48-72 | 100 48-72 |
| Human T-Cell HSB-2 | % GI Hours | 0 48-72 | 0 48-72 | 0 48-72 | 0 48-72 | 100;NVC 48;72-96 |
| Human T-Cell RPMI 8402 | % GI Hours | 0 24-96 | 0 24-96 | 0 24-96 | 0 24-96 | 100 24-96 |

| LEUKEMIA CELL TYPE | | CONCENTRATIONS μG/ML, hIFN-γ | | | | ADR μG/ML= |
|---|---|---|---|---|---|---|
| | | 0.0066 | 0.066 | 0.66 | 6.66 | 0.2 |
| Human B-Cell BALM-2 | % GI | 0;29 | 0;53;29 | 0;53 | 0;92;53 | 60;54;100 |
| | Hours | 24-72;96 | 24-48;72;96 | 24-48;72-96 | 24;48;72-96 | 24;48;72-96 |
| Human T-Cell MOLT-4 | % GI | 0 | 0 | 0 | 0 | 100 |
| | Hours | 48-96 | 48-96 | 48-96 | 48-96 | 48-96 |
| Human T-Cell HSB-2 | % GI | 0 | 0 | 0 | 0 | 100 |
| | Hours | 24-96 | 24-96 | 24-96 | 24-96 | 24-96 |
| Human T-Cell RPMI 8402 | % GI | 0 | 0 | 0 | 0 | 100 |
| | Hours | 24-96 | 24-96 | 24-96 | 24-96 | 24-96 |
| Human B-Cell SB | % GI | 0 | 0 | 0 | 0 | 100 |
| | Hours | 24-96 | 24-96 | 24-96 | 24-96 | 24-96 |
| Human B-Cell Daudi | % GI | 0 | 0 | 0;42 | 0;56;70 | 100 |
| | Hours | 24-96 | 24-96 | 24-72;96 | 24,48;72;96 | 24-96 |

| LEUKEMIA CELL TYPE | | CONCENTRATIONS PG/ML, hIFN-γ | | | | ADR, μG/ML |
|---|---|---|---|---|---|---|
| | | 0.066 | 0.66 | 2.08 | 6.66 | 0.2 |
| Human T-Cell MOLT-3 | % GI | 0 | 0 | 0 | 0 | 100 |
| | Hours | 24-96 | 24-96 | 24-96 | 24-96 | 24-96 |

NVC = No viable cells.
GI = Growth Inhibition
ADR = Adriamycin

As is apparent from the data in Tables I and II, the MOLT-3 cell line was the only T-cell line that exhibited susceptibility to both human alfa-2b interferon and human gamma interferon, whereas three B-cell lines, DND-39A, Daudi and BALM-2 exhibited sensitivity to both interferons. Since the DND-39A and Daudi cell lines exhibited reproducible replicative competency, they, along with the MOLT-3 cell line were selected for combination studies as being representative of susceptible T- and B-leukemia cell lines.

Combination Studies

The interferon concentrations were selected based on the data in Tables I and II. The concentrations selected for use in the combination studies were suboptimal in order to determine if they would exhibit an increased effectiveness when used in combination with each other.

Selected concentrations of human alfa-2b or human gamma interferons were added to proliferating B- or T-cell populations either simultaneously or sequentially as follows:

(a) human alfa-2b interferon at time zero followed by human gamma interferon at 24 hours;
(b) human gamma interferon at time zero followed by human alfa-2b interferon at 24 hours.

Human alfa-2b or human gamma interferons were separately added to additional individual cultures at time zero and at 24 hours. All test conditions for each cell line were run in parallel in the same experiment.

The results are defined as the survived fraction (SF) which is used to define the combination effects.

The equation for determining SF based on viable cell counts is as follows:

$$SF = \frac{NT_t - NC_o}{NC_t - NC_o}$$

$NT_t$ = Number of cells/ml from treated culture at 24, 48, 72 or 96 hours.
$NC_t$ = Number of cells/ml from control culture at 24, 48, 72, or 96 hours.

6

$NC_o$ =     Number of cells/ml from culture just prior to addition of agent.

The combination effects with regard to drug A (hIFN-α2b), Drug B (hIFN-γ) are based on the criteria presented and discussed by Valeriote et al., Cancer Chemother. Rep. 59 (Pt. 1) 895-900 (1975) and are defined as in the following equations:

1. No effect of a drug in a combination:

$SF_{A+B}$ = $(SF_A)$ × $(SF_B)$ and either $SF_A$ or $SF_B$ = 1.0

2. Additive effect of two drugs in a combination:

$SF_{A+B}$ = $(SF_A)$ × $(SF_B)$

3. Synergistic effect of two drugs in a combination:

$SF_{A+B}$<$(SF_A)$ × $(SF_B)$

4. Antagonistic effect of two drugs in a combination:

$SF_{A+B}$>$(SF_A)$ × $(SF_B)$ when $SF_A$ ≠ $SF_B$ ≠ $SF_{A+B}$

5. Subadditive effect of two drugs in a combination:

$SF_{A+B}$>$(SF_A)$ × $(SF_B)$ when $SF_A$ = $SF_{A+B}$<$SF_B$ or when $SF_{A+B}$ = $SF_A$ = $SF_B$

6. Interference of two drugs with each other in a combination:

$SF_{A+B}$>$(SF_A)$ × $(SF_B)$ when $SF_{A+B}$ = 1.0, $SF_A$ = 1.0, and $SF_B$<1.0 $SF_{A+B}$>$(SF_A)$ × $(SF_B)$ when $SF_{A+B}$<$SF_A$ but $SF_{A+B}$>$SF_B$

The following Table III summarizes the results of the combination treatment of leukemia cells, using recombinant human alfa-2b interferon and recombinant human gamma interferon.

TABLE III

| COMBINATION STUDIES WITH RECOMBINANT HUMAN ALFA-2b AND GAMMA INTERFERONS | | | | | |
|---|---|---|---|---|---|
| COMBINATION | LEUKEMIA CELL TYPE | COMBINATION EFFECTS | | | |
| | | 24 HR | 48 HR | 72 HR | 96 HR |
| Simultaneous Addition 0.66 μg/ml hIFN-α2b + 0.66 ng/ml hIFN-γ | Human B-Cell DND-39A | 1 | 3 | 3 | 1 |
| Sequential Addition 0.66 μg/ml hIFN-α2b at time zero + 0.66 ng/ml hIFN-γ at 24 hr | Human B-Cell DND-39A | - | 4 | 3 | 1 |
| Sequential Addition 0.66 ng/ml hIFN-γ at time zero + 0.66 μg/ml hIFN-α-2b at 24 hr | Human B-Cell DND-39A | - | 1 | 1 | 1 |
| Simultaneous Addition 0.066 ng/ml hIFN-α-2b + 6.66 μg/ml γ-IFN | Human B-Cell Daudi | 5 | 3 | 3 | 3 |
| Sequential Addition 0.066 ng/ml hIFN-α-2b at time zero + 6.66 μg/ml hIFN-γ at 24 hours | Human B-Cell Daudi | - | 4 | 1 | 1 |
| Sequential Addition 6.66 μg/ml hIFN-γ at time zero + 0.066 ng/ml hIFN-α2b | Human B-Cell Daudi | - | 1 | 4 | 1 |
| Simultaneous Addition 6.66 ng/ml hIFN-α2b + 6.66 ng/ml hIFN-γ | Human T-Cell MOLT-3 | - | 5 | 2 | 1 |
| Sequential Addition 6.66 ng/ml hIFN-α2b at time zero + 6.66 ng/ml hIFN-γ at 24 hours | Human T-Cell MOLT-3 | - | 5 | 2 | 1 |
| Sequential Addition 6.66 ng/ml hIFN-γ at time zero + 6.66 ng/ml hIFN-α2b at 24 hours | Human T-Cell MOLT-3 | - | 5 | 2 | 6 |

In Table III the combination effects are presented using the number code 1 through 6 for determining combination effects as discussed supra.

As is apparent from the data in Table III synergistic effects were found after 48 and 72 hours upon the simultaneous addition of 0.66 μg/ml of hIFN-α2b and 0.66 ng/ml of hIFN-γ against human B-cell DND-39A, and 0.066 ng/ml of hIFN-α2b and 6.66 μg/ml hIFN-γ at 48, 72 and 96 hours against Human B-Cell Daudi.

Synergism was found after 72 hours upon sequential addition of 0.66 μg/ml of hIFN-α2b at time zero and 0.66 ng/ml of hIFN-γ at 24 hours.

Additive effects were found after 72 hours upon simultaneous addition of 6.66 ng/ml of hIFN-α2b and 6.66 ng/ml of hIFN-γ against Human T-Cell MOLT-3; sequential addition of 6.66 ng/ml of hIFN-α2b at time zero and 6.66 ng/ml hIFN-γ at 24 hours against Human T-Cell MOLT-3 and sequential addition of 6.66 ng/ml of hIFN-γ at time zero and 6.66 ng/ml of hIFN-α2b at 24 hours.

Although the data indicate that with the two B-cell leukemias, the most effective schedule is simultaneous addition of recombinant human alfa-2b and gamma interferons at time zero, and no synergism was observed at the concentrations and schedule for addition used in experiments with the T-cell leukemia, however, because of additive effects observed, it is expected that adjusting the concentrations and schedule might result in synergistic effects.

As is apparent from the above, each T-cell and B-cell line have different sensitivity to the combination treatment and each patient's cells must be tested to determine susceptibility to the treatment and the concentrations of the interferons used as well as the treatment regimen. Thus, the dosages of recombinant human alpha interferon and recombinant human gamma interferon when used in combination to treat susceptible leukemia cells, as well as the regimen and form of administration, are dependent on the judgment of the clinician, taking into account a variety of factors including the patient's leukemia cells response to the combination using the tests described.

Recombinant human alfa-2b interferon and recombinant human gamma interferon are each preferably administered parenterally, e.g. intravenously, intramuscularly, subcutaneously or intraperitoneally in conventional dosage forms, i.e. sterile aqueous solutions or suspensions. Pharmaceutically acceptable parenteral compositions can be prepared, for example, by diluting the interferons with sterile pyrogen free water for injection to produce sterile solutions containing the appropriate concentrations of the interferons and if desired other conventional pharmaceutically acceptable suitable carriers, adjuvants, fillers, binders, disintegrants or buffers.

## Claims

1. The use of recombinant human alpha interferon in the manufacture of a pharmaceutical composition for use in inhibiting the proliferation of susceptible human lymphoma derived B cell leukemia cells by a combination therapy involving simultaneously or sequentially treating such cells with an effective amount of a combination of recombinant human alpha interferon and recombinant human gamma interferon.

2. The use of recombinant human gamma interferon in the manufacture of a pharmaceutical composition for use in inhibiting the proliferation of susceptible human lymphoma derived B cell leukemia cells by a combination therapy involving simultaneously or sequentially treating such cells with an effective amount of a combination of recombinant human alpha interferon and recombinant human gamma interferon.

3. The use according to claim 1 wherein said alpha interferon and said gamma interferon are administered sequentially.

4. The use according to claim 1 wherein said alpha interferon and said gamma interferon are administered simultaneously.

5. The use according to claim 2 wherein said alpha interferon and said gamma interferon are administered sequentially.

6. The use according to claim 2 wherein said alpha interferon and said gamma interferon are administered simultaneously.

7. The use according to any of claims 1 to 6 wherein said alpha interferon is recombinant human alpha-2b.

## Revendications

1. Utilisation de l'interféron alpha humain recombinant dans la fabrication d'une composition pharmaceutique à utiliser pour l'inhibition de la prolifération des cellules des cellules B sensibles dérivées d'un lymphome humain par une thérapie en association impliquant le traitement simultané ou séquentiel de telles cellules par une quantité efficace d'une combinaison d'interféron alpha humain recombinant et d'interféron gamma humain recombinant.

2. Utilisation de l'interféron gamma humain recombinant dans la fabrication d'une composition pharmaceutique à utiliser pour l'inhibition de la prolifération des cellules de leucémie des cellules B sensibles

8

dérivées du lymphome humain par une thérapie en association impliquant le traitement simultané ou séquentiel de telles cellules par une quantité efficace d'une association d'un interféron alpha humain recombinant et d'un interféron gamma humain recombinant.

**3.** Utilisation selon la revendication 1 où ledit interféron alpha et ledit interféron gamma sont administrés séquentiellement.

**4.** Utilisation selon la revendication 1 où ledit interféron alpha et ledit interféron gamma sont administrés simultanément.

**5.** Utilisation selon la revendication 2 où ledit interféron alpha et ledit interféron gamma sont administrés séquentiellement.

**6.** Utilisation selon la revendication 2 où ledit interféron alpha et ledit interféron gamma sont administrés simultanément.

**7.** Utilisation selon l'une quelconque des revendications 1 à 6 où ledit interféron alpha est alpha-2b humain recombinant.

**Patentansprüche**

**1.** Verwendung von rekombinantem Human-alpha-Interferon bei der Herstellung einer pharmazeutischen Zusammensetzung zur Verwendung bei der Hemmung der Proliferation suszeptibler, von Human-Lymphom stammender B-Zellen-Leukämie-Zellen durch eine Kombinationstherapie, bei der solche Zellen gleichzeitig oder nacheinander mit einer wirksamen Menge einer Kombination von rekombinantem Human-alpha-Interferon und rekombinantem Human-gamma-Interferon behandelt werden.

**2.** Verwendung von rekombinantem Human-gamma-Interferon bei der Herstellung einer pharmazeutischen Zusammensetzung zur Verwendung bei der Hemmung der Proliferation suszeptibler, von Human-Lymphom stammender B-Zellen-Leukämie-Zellen durch eine Kombinationstherapie, bei der solche Zellen gleichzeitig oder nacheinander mit einer wirksamen Menge einer Kombination von rekombinantem Human-alpha-Interferon und rekombinantem Human-gamma-Interferon behandelt werden.

**3.** Verwendung nach Anspruch 1, worin das alpha-Interferon und das gamma-Interferon nacheinander verabreicht werden.

**4.** Verwendung nach Anspruch 1, worin das alpha-Interferon und das gamma-Interferon gleichzeitig verabreicht werden.

**5.** Verwendung nach Anspruch 2, worin das alpha-Interferon und das gamma-Interferon nacheinander verabreicht werden.

**6.** Verwendung nach Anspruch 2, worin das alpha-Interferon und das gamma-Interferon gleichzeitig verabreicht werden.

**7.** Verwendung nach irgendeinem der Ansprüche 1 bis 6, worin das alpha-Interferon rekombinantes Human-alpha-2b ist.